# EUROPÄISCHE PATENTSCHRIFT

(11) **EP 2 086 411 B1**
(45) Veröffentlichungstag und Bekanntmachung des Hinweises auf die Patenterteilung: **11.11.2015**
(21) Anmeldenummer: 07803264.6
(22) Anmeldetag: 05.09.2007
(51) Int. Cl.: B60W 50/14, B60W 40/09, A61B 5/18

(54) **FAHRERINFORMATIONS- UND -DIALOGSYSTEM**
DRIVER INFORMATION AND DIALOGUE SYSTEM
SYSTÈME D'INFORMATION ET DE DIALOGUE D'UN CONDUCTEUR

(30) Priorität: 03.11.2006 DE 102006051922
(43) Veröffentlichungstag der Anmeldung: 12.08.2009
(73) Patentinhaber: Robert Bosch GmbH, 70442 Stuttgart (DE)
(72) Erfinder: KOENIG, Winfried, 76327 Pfinztal (DE)
(86) Internationale Anmeldenummer: PCT/EP2007/059302
(87) Internationale Veröffentlichungsnummer: WO 2008/052829

(56) Entgegenhaltungen:
- US-A- 4 706 072
- US-A1- 2002 109 602
- US-B1- 6 952 161

## Beschreibung

Die Erfindung betrifft ein Fahrerinformations- und -dialogsystem nach Oberbegriff des Anspruchs 1.

### Stand der Technik

Im Stand der Technik sind Beobachtungssysteme bekannt, die Daten über das Verhalten des Fahrers und/oder Daten über den Zustand des Fahrers von Kraftfahrzeugen erfassen. Solche Daten sind beispielsweise das Fahrverhalten des Fahrers, die gefahrene Strecke, der Zustand des Fahrers, seine Gestik, Mimik et cetera. Weiter werden Erfassungssysteme, die die Dynamik des Fahrzeugs erfassen, Daten von Zielführungssystemen, von Assistenzsystemen und von bildgebenden Sensoren ausgewertet. Diese Systeme dienen im weitesten Sinne dazu, mögliche Fahrfehler des Fahrers frühzeitig zu erkennen und auszuschalten oder den Fahrer vor möglichen Fahrfehlern zu warnen und/oder den Fahrer auf die Fahrsicherheit negativ beeinflussende Umstände, wie beispielsweise Übermüdung, aufmerksam zu machen. Die Datenerfassung und Datenaufbereitung ist außerordentlich komplex. Die Speicherung von solcher Art erfassten Daten erfolgt häufig im Fahrzeug, beispielsweise zur Dokumentation für Gewährleistungsfragen, teilweise werden Daten auch nach Außen übertragen. Aufgrund der Komplexität der Systeme ist es für den Fahrer regelmäßig nicht möglich zu erfahren, welche Daten erfasst werden, wie diese in Bezug zu seinem Verhalten stehen, welche davon abgespeichert werden und wie diese Daten weiterverarbeitet werden.

Aus den Offenlegungsschriften US 4,706,072 A und US 6,952,161 B1 sind Systeme bekannt, die Anomalien im Verhalten eines Fahrers erfassen und dies dem Fahrer gegebenenfalls anzeigen. Dabei hat der Fahrer auch die Möglichkeit, das System zu deaktivieren.

### Offenbarung der Erfindung

Aufgabe der Erfindung ist es, dem Fahrer auf leicht verständliche und klare Art und Weise eine Entscheidung darüber zu ermöglichen, welche Daten erfasst, gespeichert und/oder Anderen zur Verfügung gestellt werden.

Hierzu wird ein in einem Fahrzeug angeordnetes Fahrerinformations- und -dialogsystem betreffend Fahrverhaltens- und -zustandsdaten vorgeschlagen, mit einer Datenerfassungseinrichtung und einer Ein-/Ausgabeeinrichtung, wobei die Ausgabeeinrichtung den Fahrer über ein Erheben, Speichern, Verwenden, Verarbeiten und/oder Weitergeben von Fahrerverhaltens- und -zustandsdaten informiert und die Eingabeeinrichtung dem Fahrer zur Abgabe seiner zustimmenden oder ablehnenden Fahrerentscheidung über das Erheben, Speichern, Verwenden, Verarbeiten und/oder Weitergeben von Fahrerverhaltens- und -zustandsdaten dient. Die bislang für den Fahrer des Fahrzeugs vorhandene Undurchsichtigkeit des Systems der "Blackbox" wird demzufolge zugunsten einer freien Entscheidung des Fahrers durchbrochen, wobei dieser selbst hinsichtlich von Fahrerverhaltens- und -zustandsdaten entscheiden kann, ob diese überhaupt erhoben werden, ob sie gegebenenfalls gespeichert werden, verwendet, verarbeitet und/oder weitergegeben werden. Der Fahrer behält hierbei die vollständige Kontrolle über das System.

In einer bevorzugten Ausführungsform erfasst die Datenerfassungseinrichtung Daten von einem Zielführungssystem, mindestens einem Fahrzustandssensor, mindestens einem Fahrerassistenzsystem, einer Fahrerbeobachtungssensorik und/oder einer Fahrerzustandssensorik.

In einem weiteren bevorzugten Ausführungssystem werden die erfassten Daten einem Auswertungssystem zugeführt. Das Auswertungssystem setzt die erfassten Daten zueinander in Bezug und wertet sie nach vorgegebenen Kriterien aus.

Die Erfindung weist außerdem ein Datenübertragungssystem zum Datenaustausch mit mindestens
einer stationären Einrichtung auf. Die vom Fahrer genehmigten Daten werden hier, gegebenenfalls erst bei Bedarf, an eine stationäre Einrichtung übertragen. Gleichzeitig kann es ebenfalls möglich sein, an die stationäre Einrichtung Daten zu übertragen, soweit dies erforderlich und/oder gewünscht ist.

In einer weiteren bevorzugten Ausführungsform ist vorgesehen, dass die Ausgabeeinrichtung dem Fahrer in Abhängigkeit mindestens eines Auswerteergebnisses des Auswertungssystems Fahrerverhaltens- und/oder Fahrerzustandsinformationen liefert und/oder Vorschläge unterbreitet. In dieser Ausführungsform muss der Fahrer nicht blind über die Erfassung von Daten beziehungsweise deren weitere Verwendung, beispielsweise Speicherung, entscheiden, sondern kann dies in Abhängigkeit mindestens eines bereits vorhandenen Auswerteergebnisses tun, wobei ihm in einer besonders bevorzugten Ausführungsform das Fahrerinformations- und -dialogsystem auch Vorschläge unterbreitet. Beispielsweise ist es hierbei möglich, dem Fahrer in klar verständlicher Form eine Information über die im Fahrzeug erhobenen, die gespeicherten und eventuell zur externen Stellen zu übertragenden Daten zu geben, diese in Abhängigkeit bereits erfasster Daten zu klassifizieren und dem Fahrer anhand des Ergebnisses sachdienliche Vorschläge zu unterbreiten.

Die Erfindung betrifft ferner ein Verfahren zur Fahrerinformation und zum Fahrerdialog bezüglich in einem Fahrzeug erhobener Fahrerverhaltens- und -zustandsdaten. Es ist vorgesehen, dass der Fahrer über das Erheben, Speichern, Verwenden, Verarbeiten und/oder Weitergeben von Fahrerverhaltens- und -zustandsdaten informiert wird und der Fahrer über das Erheben, Speichern, Verwenden, Verarbeiten und/oder Weitergeben der Fahrerverhaltens- und -zustandsdaten eine Fahrerentscheidung trifft. Abweichend von dem Stand der Technik bekannten Verfahren zur Erhebung solcher Daten wird dem Fahrer hinsichtlich jedes Einzeldatums beziehungsweise von Datenklassen die Entscheidung gestattet, ob diese überhaupt erhoben, gespeichert, verwendet, verarbeitet und/oder weitergegeben werden sollen.

### Kurze Beschreibung der Zeichnungen

Die Erfindung wird nachfolgend anhand von Zeichnungen näher erläutert. Dazu zeigt die
Figur 1 eine schematische Darstellung eines Fahrerinformations- und -dialogsystems und
Figur 2 einen Dialog- und Entscheidungspfad zum Treffen einer verfahrensgemäßen Fahrerentscheidung.

### Ausführungsform(en) der Erfindung

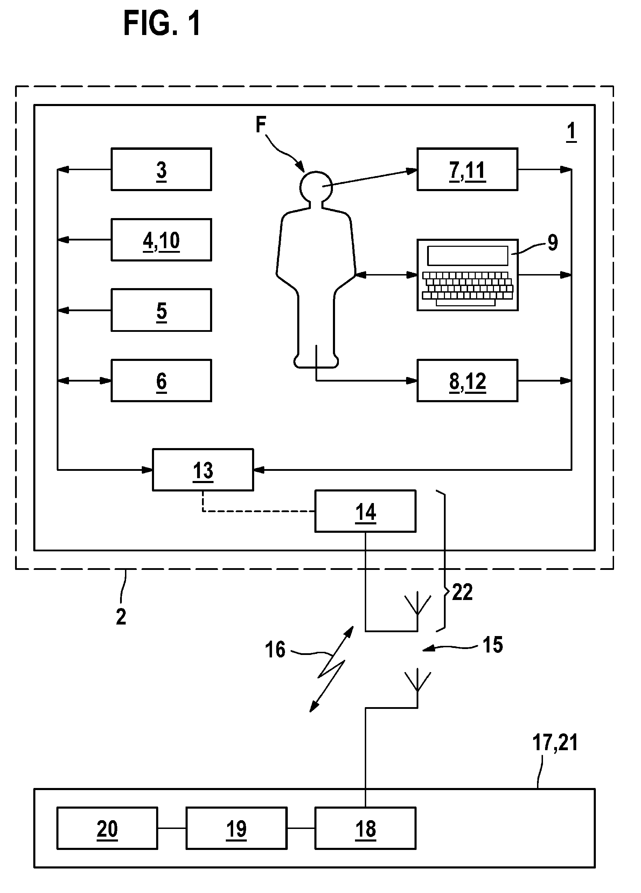

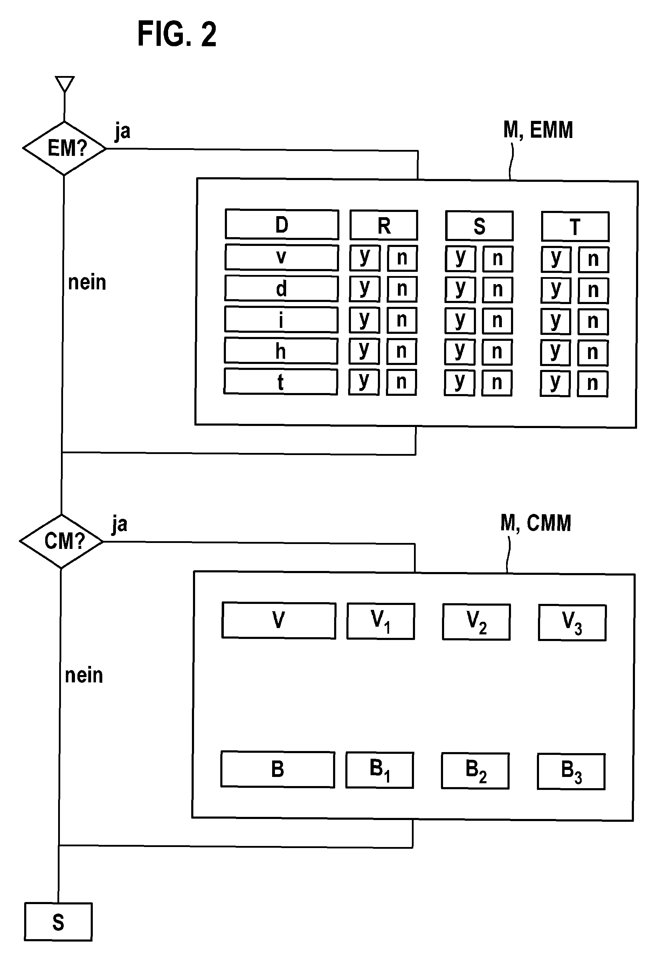

Figur 1 zeigt ein Fahrerinformations- und -dialogsystem 1 in schematischer Darstellung/Blockdarstellung eines Kraftfahrzeugs 2. Das Fahrerinformations- und - dialogsystem 1 umfasst ein Zielführungssystem 3, Dynamiksensoren 4, Fahrerassistenzsysteme 5 sowie einen Datenspeicher 6 zur Datenspeicherung von Daten des Zielführungssystems 3, der Dynamiksensoren 4 und der Fahrerassistenzsysteme 5 innerhalb des Fahrzeugs. Das Fahrerinformations- und -dialogsystem 1 umfasst weiter eine bildgebende Sensorik 7, eine Fahrerparametererfassung 8 und eine Ein-/Ausgabeeinrichtung 9. Der Dynamiksensor 4 ist hierbei insbesondere als Fahrzustandssensor 10 ausgebildet, die bildgebende Sensorik 7 als Fahrerbeobachtungssensorik 11 und die Fahrerparametererfassung 8 als Fahrerzustandssensorik 12. Der Datenaustausch der einzelnen Komponenten erfolgt teils bidirektional, insbesondere hinsichtlich des Datenspeichers 6 und der Ein-/Ausgabeeinrichtung 9. Die Daten fließen in einem Beobachtungssystem 13 zusammen, dem ein Sender/Empfänger 14 als Datenübertragungssystem 22 zum Datenaustausch über eine Luftschnittstelle 15, also insbesondere über Funk 16 beispielsweise zu einer stationären Überwachungszentrale 17 angeschlossen ist, in der die Daten wiederum von einer Sende-/Empfangseinheit 18 empfangen und mittels eines Rechners 19 verarbeitet sowie in einem Stationärdatenspeicher 20 gespeichert werden. Die stationäre Überwachungszentrale als stationäre Einrichtung 21 ist hierbei keine zwingende Voraussetzung für das Fahrerinformations- und -dialogsystem 1, ihr Vorhandensein macht aber das Vorhandensein eines Fahrerinformations- und -dialogsystems im Kraftfahrzeug 2 für einen Fahrer F wünschenswert.

Figur 2 zeigt einen Dialog- und Entscheidungspfad zum Treffen einer verfahrensgemäßen Fahrerentscheidung. In einer Darstellung der hier nicht dargestellten Ein-/Ausgabeeinrichtung 9 erhält der Fahrer F die nachfolgend aufgezeigte Informations- und Entscheidungsstruktur: Der Fahrer F wird aufgefordert zu entscheiden, ob er die Fahrerentscheidung in einem Expertenmodus EM oder in einem Komfortmodus CM treffen möchte, wobei der Komfortmodus CM immer dann eingeschaltet wird, wenn der Fahrer F nicht explizit den Expertenmodus EM wählt. Alternativ hierzu ist es möglich, das System ganz abzuschalten oder eine Erhebung oder wie auch immer geartete Weiterverarbeitung von Daten vollständig zu unterbinden, sofern weder der Expertenmodus EM noch der Komfortmodus CM gewählt wird; diese hier im Verfahrensablauf als Stop S bezeichnete Verfahrensposition kann mit für die Ausführung des Verfahrens und in Abhängigkeit vom Fahrerinformations- und -dialogsystem gewählten beziehungsweise ausgestalteten weiteren Entscheidungsstrukturen oder Aktionen besetzt werden. Der Expertenmodus EM und der Komfortmodus CM unterscheiden sich durch die dem Fahrer F zum Zweck der Information und Ausübung der Fahrerentscheidung dargestellten Informations- und Entscheidungsstruktur, der Matrix M, die im Expertenmodus als Expertenmodus-Matrix EMM und im Komfortmodus als Komfortmodus-Matrix CMM ausgebildet ist. In der Expertenmodus-Matrix EMM werden dem Fahrer F weitaus mehr Entscheidungsmöglichkeiten angeboten als in der Komfortmodus-Matrix CMM, in der die Entscheidungen im Wesentlichen auf hier beispielsweise zwei Typklassen reduziert sind, die dem Fahrer F je nach Kategorisierung eine besonders komfortable Einstellung ermöglichen. Er entscheidet innerhalb der Komfortmodus-Matrix CMM lediglich über den Grad an Vertraulichkeit V und darüber, ob das Fahrerinformations- und -dialogsystem 1 ihn zum verkehrssicheren Fahren beraten soll, also über den Umfang der vom Fahrerinformations- und -dialogsystem 1 zu leistenden Beratung B. Innerhalb der Vertraulichkeit V entscheidet der Fahrer F über die Stufe der Vertraulichkeit, die als geringe Vertraulichkeit V₁, als mittlere Vertraulichkeit V₂ und als hohe Vertraulichkeit V₃ ausgeprägt sein kann, wobei entsprechend der gewählten Vertraulichkeitsstufe die von dem Fahrerinformations- und -dialogsystem 1 zu erhebenden, speichernden, verwendenden, verarbeitenden und/oder weiterzugebenden Fahrerverhaltens- und -zustandsdaten durch das Fahrerinformations- und -dialogsystem selbsttätig entsprechend der vorgenommenen Einstufung und Kategorisierung ausgewählt werden. Beispielsweise ist es in der Stufe hohe Vertraulichkeit V₃ dem Fahrerinformations- und -dialogsystem 1 nicht gestattet, Fahrerverhaltens- und -zustandsdaten weiterzugeben, insbesondere über eine Luftschnittstelle 15 an eine stationäre Überwachungszentrale 17 weiterzugeben. Entscheidet sich der Fahrer F in seiner Fahrerentscheidung hingegen für geringe Vertraulichkeit V₁, ist das Erheben, Speichern, Verwenden, Verarbeiten und/oder Weitergeben von Fahrerverhaltens- und -zustandsdaten in vollem Umfange gestattet. Weiter kann der Fahrer F eine Entscheidung über die von dem Fahrerinformations- und -dialogsystem gewünschten Beratung B betreffend verkehrssicheres Fahren in Abhängigkeit von den erfassten Fahrerverhaltens- und -zustandsdaten treffen, wobei er zwischen beispielsweise den Stufen "keine Beratung" B1, "tolerante Beratung" B2 und "genaue Beratung" B3 wählen kann. Abhängig vom Grad der gewünschten Beratungsintensität B1, B2 oder B3 erteilt das Fahrerinformations- und -dialogsystem 1 in Abhängigkeit von den erfassten Fahrerverhaltens- und -zustandsdaten Ratschläge und Hinweise, die die Fahrsicherheit des Fahrers betreffen und diese erhöhen sollen. Insbesondere ist es hierbei vorgesehen, auf Ermüdungserscheinungen des Fahrers F und unkonzentrierte Fahrweisen hinzuweisen. In einer Stufe, in der keine Beratung B1 gewählt ist, werden dem Fahrer F keine entsprechenden Hinweise und Vorschläge unterbreitet. In einer Stufe, in der tolerante Beratung B2 gewünscht ist, wird der Fahrer F auf beispielsweise körperliche Müdigkeit und die Gefahr von Ausfällen hingewiesen (beispielsweise zur Vermeidung von Sekundenschlaf), wohingegen etwa in der Stufe, in der genaue Beratung B3 gewünscht ist, der Fahrer F über eine Vielzahl von vom Fahrerinformations- und -dialogsystem 1 erfassten und verarbeiteten Daten abhängigen, beispielsweise auch psychologischen Faktoren, hingewiesen werden kann, die etwa auch aggressiven Fahrstil, Ärger et cetera umfassen, wobei diese Fahrerzustände in komplexer Weise aus einer Vielzahl von Fahrerverhaltens- und -zustandsdaten vom Fahrerinformations- und -dialogsystem gewonnen und ermittelt werden.

Im Expertenmodus EM hat der Fahrer F die Möglichkeit, in der Expertenmodus-Matrix EMM eine differenzierte Entscheidung zu treffen. Hierzu werden ihm Auswahlmöglichkeiten betreffend Daten D über die Geschwindigkeit v seines Fahrzeugs, den Abstand zum vorausfahrenden Fahrzeug d, zur Spurhaltung i, zu seinem Kreislauf h und zum Grad seiner Müdigkeit t präsentiert. Diese Daten D werden vom Fahrerinformations- und -dialogsystem über insbesondere einer Fahrerbeobachtungssensorik 11 und/oder eine Fahrerzustandssensorik 12 erfasst, hinzu kommen weitere Parameter von weiteren fahrzeugüblichen Sensoren. Hinsichtlich eines jeden Datums D ist eine Auswahlmöglichkeit über das Aufnehmen beziehungsweise Erfassen R, das Speichern S und über das Weitergeben T gegeben. Innerhalb der Entscheidungsgruppen zum Aufnehmen/Erfassen R, zum Speichern S und zum Weitergeben T kann hinsichtlich jedes Datums betreffend die Geschwindigkeit v, den Abstand zum vorausfahrenden Fahrzeug d, die Spurhaltung i, den Kreislauf h und die Müdigkeit t des Fahrers F vom Fahrer F mit Ja y oder Nein n entschieden werden. Im Gegensatz zum Komfortmodus, der in der Komfortmodus-Matrix CMM die entscheidungsrelevanten Daten gruppiert und klassifiziert, und lediglich nach Stufe der Vertraulichkeit V und nach Stufe der gewünschten Beratung B entscheiden lässt, kann der Fahrer F im Expertenmodus EM innerhalb der Expertenmodus-Matrix EMM eine differenzierte Entscheidung treffen, wonach beispielsweise das Aufnehmen aller Daten betreffend Geschwindigkeit v, Abstand zum vorausfahrenden Fahrzeug d, Spurhaltung i, Kreislauf h und Müdigkeit t erfasst werden können (innerhalb des Blockes über die Aufnahme/des Erfassens R sind sämtliche Auswahlen mit Ja y getätigt), der Fahrer wünscht jedoch hinsichtlich der Geschwindigkeit v, des Abstands zum vorausfahrenden Fahrzeugs d und zu seiner Müdigkeit t keine Speicherung S und wählt hier folgerichtig Nein n. Hinsichtlich aller im Block Aufnehmen/Speichern R ausgewählten Daten D wünscht der Fahrer F keine Übermittlung an stationäre Einrichtungen 21, so dass er im Block Weitergabe T hinsichtlich aller Daten T Nein n auswählt.

Die Ausgestaltung der jeweiligen Matrix M sowohl im Expertenmodus EM als im Komfortmodus CM kann je nach den an das System zu stellenden Anforderungen und die dem Fahrer F zu gewährenden Wahlfreiheiten selbstverständlich modifiziert werden, wobei alle in Betracht kommenden, vom Fahrerinformations- und -dialogsystem zu erfassenden und verarbeitenden Daten präsentiert werden.

## Patentansprüche

1. Fahrerinformations- und -dialogsystem (1) eines Kraftfahrzeugs zum Erheben, Speichern, Verwenden, Verarbeiten und/oder Weitergeben von Fahrerverhaltens- und - zustandsdaten, mit einer Ein-/Ausgabeeinrichtung (9), wobei die Ausgabeeinrichtung (9) den Fahrer (F) über das Erheben, Speichern, Verwenden, Verarbeiten und/oder Weitergeben von Fahrerverhaltens- und -zustandsdaten informiert und die Eingabeeinrichtung (9) dem Fahrer (F) zur Abgabe seiner zustimmenden oder ablehnenden Fahrerentscheidung über das Erheben, Speichern, Verwenden, Verarbeiten und/oder Weitergeben von Fahrerverhaltens- und -zustandsdaten dient, **gekennzeichnet durch** ein Datenübertragungssystem (22) zum Datenaustausch mit mindestens einer stationären Einrichtung (21), wobei das Fahrerinformations- und -dialogsystem dazu eingerichtet ist, die vom Fahrer **durch** seine Zustimmung genehmigten Daten, gegebenenfalls erst bei Bedarf, **durch** das Datenübertragungssystem (22) an die stationäre Einrichtung (21) zu übertragen

2. Fahrerinformations- und -dialogsystem nach Anspruch 1, **dadurch gekennzeichnet, dass** eine Datenerfassungseinrichtung des Fahrerinformations- und -dialogsystem Daten (D) von einem Zielführungssystem (3), mindestens einem Fahrzustandssensor (10), mindestens einem Fahrerassistenzsystem (5), Fahrerbeobachtungssensorik (11) und/oder Fahrerzustandssensorik (12) erfasst.

3. Fahrerinformations- und -dialogsystem nach einem der vorhergehenden Ansprüche, **dadurch gekennzeichnet, dass** die erfassten Daten (D) einem Auswertungssystem zugeführt werden.

4. Fahrerinformations- und -dialogsystem nach einem der vorhergehenden Ansprüche, **dadurch gekennzeichnet, dass** die Ausgabeeinrichtung (9) dem Fahrer (F) in Abhängigkeit mindestens eines Auswerteergebnisses des Auswertungssystems Fahrerverhaltens- und/oder Fahrerzustandsinformationen liefert und/oder Vorschläge unterbreitet.

5. Verfahren zur Fahrerinformation und zum Fahrerdialog bezüglich in einem Fahrzeug erhobener Fahrerverhaltens- und -zustandsdaten, wobei der Fahrer (F) über das Erheben, Speichern, Verwenden, Verarbeiten und/oder Weitergeben von Fahrerverhaltens- und -zustandsdaten informiert wird und der Fahrer (F) über das Erheben, Speichern, Verwenden, Verarbeiten und/oder Weitergeben der Fahrerverhaltens- und -zustandsdaten eine zustimmende oder ablehnende Fahrerentscheidung trifft, **dadurch gekennzeichnet, dass** die vom Fahrer durch seine Zustimmung genehmigten Daten, gegebenenfalls erst bei Bedarf, an eine stationäre Einrichtung übertragen werden.

## Claims

1. Driver information and dialogue system (1) in a motor vehicle for collecting, storing, using, processing and/or forwarding driver behaviour and condition data, having an input/output device (9), wherein the output device (9) informs the driver (F) about the collection, storage, use, processing and/or forwarding of driver behaviour and condition data and the input device (9) can be used by the driver (F) to output his consenting or declining driver decision about the collection, storage, use, processing and/or forwarding of driver behaviour and condition data, **characterized by** a data transmission system (22) for data interchange with at least one static device (21), wherein the driver information and dialogue system is set up to transmit the data that the driver has approved through his consent, if need be only when required, to the static device (21) using the data transmission system (22).

2. Driver information and dialogue system according to Claim 1, **characterized in that** a data acquisition device of the driver information and dialogue system acquires data (D) from a route planning system (3), at least one driving condition sensor (10), at least one driver assistance system (5), driver observation sensors (11) and/or driver condition sensors (12).

3. Driver information and dialogue system according to either of the preceding claims, **characterized in that** the acquired data (D) are supplied to an evaluation system.

4. Driver information and dialogue system according to one of the preceding claims, **characterized in that** the output device (9) provides the driver (F) with driver behaviour and/or driver condition information, and/or submits proposals, on the basis of at least one evaluation result from the evaluation system.

5. Method for driver information and for driver dialogue in respect of driver behaviour and condition data collected in a vehicle, wherein the driver (F) is informed about the collection, storage, use, processing and/or forwarding of driver behaviour and condition data and the driver (F) makes a consenting or declining driver decision about the collection, storage, use, processing and/or forwarding of the driver behaviour and condition data, **characterized in that** the data that the driver has approved through his consent are transmitted, if need be only when required, to a static device.

## Revendications

1. Système d'information et de dialogue d'un conducteur (1) d'un véhicule automobile destiné à collecter, stocker, utiliser, traiter et/ou transmettre des données de comportement et d'état du conducteur, comportant un dispositif d'entrée/sortie (9), dans lequel le dispositif de sortie (9) informe le conducteur (F) de la collecte, du stockage, de l'utilisation, du traitement et/ou de la transmission de données de comportement et d'état du conducteur et le dispositif d'entrée (9) sert au conducteur (F) à fournir sa décision acceptée ou refusée en ce qui concerne la collecte, le stockage, l'utilisation, le traitement et/ou la transmission de données de comportement et d'état du conducteur, **caractérisé par** un système de transmission de données (22) destiné à échanger des données avec au moins un dispositif stationnaire (21), dans lequel le système d'information et de dialogue du conducteur est conçu pour ne transmettre le cas échéant les données acceptées conformément à son accord, qu'en cas de besoin, par le système de transmission de données (22) au dispositif stationnaire (21).

2. Système d'information et de dialogue d'un conducteur selon la revendication 1, **caractérisé en ce qu'**un dispositif de détection de données du système d'information et de dialogue du conducteur détecte des données (D) provenant d'un système de guidage routier (3), d'au moins un capteur d'état de conduite (10), d'au moins un système d'aide à la conduite (5), de capteurs d'observation du conducteur (11) et/ou de capteurs d'état du conducteur (12).

3. Système d'information et de dialogue d'un conducteur selon l'une quelconque des revendications précédentes, **caractérisé en ce que** les données détectées (D) sont envoyées à un système d'évaluation.

4. Système d'information et de dialogue d'un conducteur selon l'une quelconque des revendications précédentes, **caractérisé en ce que** le dispositif de sortie (9) délivre au conducteur (F) en fonction d'au moins un résultat d'évaluation du système d'évaluation, des informations de comportement du conducteur et/ou d'état du conducteur et/ou fournit des suggestions.

5. Procédé d'information d'un conducteur et de dialogue d'un conducteur concernant des données de comportement et d'état d'un conducteur collectées dans un véhicule, dans lequel le conducteur (F) est informé de la collecte, du stockage, de l'utilisation, du traitement et/ou de la transmission de données de comportement et d'état du conducteur et le conducteur (F) prend une décision de conduite acceptée ou refusée en ce qui concerne la collecte, le stockage, l'utilisation, le traitement et/ou la transmission des données de comportement et d'état du conducteur, **caractérisé en ce que** les données autorisées conformément à son accord ne sont transmises le cas échéant à un dispositif stationnaire qu'en cas de besoin.
